(19) Europäisches Patentamt
European Patent Office
Office européen des brevets

(11) EP 4 024 344 A1

(12) EUROPEAN PATENT APPLICATION

(43) Date of publication:
06.07.2022 Bulletin 2022/27

(21) Application number: 21212677.5

(22) Date of filing: 07.12.2021

(51) International Patent Classification (IPC):
G06T 17/20 (2006.01)    G06T 19/00 (2011.01)

(52) Cooperative Patent Classification (CPC):
G06T 17/205; G06T 19/00; G06T 2210/41

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 08.12.2020 US 202017115199

(71) Applicant: Biosense Webster (Israel) Ltd
Yokneam, 2066717 (IL)

(72) Inventors:
• MONTAG, Avram Dan
  2066717 Yokneam (IL)
• BAR-TAL, Meir
  2066717 Yokneam (IL)

(74) Representative: Carpmaels & Ransford LLP
One Southampton Row
London WC1B 5HA (GB)

(54) **SIGNAL PROCESSING OF VELOCITY STREAMS OF A SIGNAL FLOW FOR COHERENT MAPPING OF AN ANATOMICAL STRUCTURE**

(57)    A method is provided. The method is implemented by a mapping engine stored as program code on a memory and executed by a processor. The method include subdividing an anatomical mesh of a part of an anatomical structure to one or more other meshes of the anatomical structure. The one or more other meshes are more granular than the anatomical mesh. The method includes interpolating local activation time values and velocity values for the one or more other meshes and tracing a path of velocity vectors on the one or more other meshes in accordance with the interpolation of the local activation time values and velocity values. The method also includes projecting the path on the anatomical mesh to provide an enhanced visualization of the anatomical structure.

FIG. 6

EP 4 024 344 A1

**Description**

FIELD OF INVENTION

The present invention is related to signal processing. More particularly, the present invention relates to signal correction processing of velocity streams of a signal flow for coherent mapping of an anatomical structure.

BACKGROUND

In medical procedures, such as mapping electrical activity of an organ (e.g., a heart), physicians use current medical mapping systems to measure and track a flow and progression of signals around the heart.

In general, when mapping electrical activity, the current medical mapping systems indicate a correspondence between a set of local activation times (LATs) and a group of spatial map elements, such a triangles (e.g., as discussed in U.S. Application No. 2020/0146579, which is incorporated herein by reference. LATs are indications of a flow of electrical activity through walls of the heart, associated with a beating of the heart. The spatial map elements (e.g., such as triangles) may be generated from measured positions of the heart wall. Note that current medical mapping systems can show correspondences between the LATs and the triangles in a mesh form, superimposed on a graphic representation of the wall of the heart, as the mapping. To refine this mesh form and mapping, current medical mapping systems perform interpolations with respect to the mesh form imposed on the wall of the heart and by calculation of velocity vectors for an electrical wave on the mesh form (e.g., for each triangle, a signal arrival time and a signal velocity is calculated).

In particular, a coherent mapping feature in the current medical mapping systems present a clear visualization of the flow of a signal in the heart, based on the color-coded indication of a progress of the signal. The coherent mapping feature also provides a time and velocity for the travel of the signal around the heart, especially for cyclic arrhythmias. However, when physicians attempt to understand the flow of the arrhythmias, physicians must analyze velocity vectors for any given triangle, which may be difficult to interpret because the coarseness of the mesh over which the velocity vectors were determined may appear as discontinuous, even though the activation progress is smooth.

SUMMARY

According to an embodiment, a method is provided. A method is provided. The method is implemented by a mapping engine stored as program code on a memory and executed by a processor. The method include subdividing an anatomical mesh of a part of an anatomical structure to one or more other meshes of the anatomical structure. The one or more other meshes are more granular than the anatomical mesh. The method includes interpolating local activation time values and velocity values for the one or more other meshes and tracing a path of velocity vectors on the one or more other meshes in accordance with the interpolation of the local activation time values and velocity values. The method also includes projecting the path on the anatomical mesh to provide an enhanced visualization of the anatomical structure.

According to one or more embodiments, the method embodiment above can be implemented as an apparatus, a system, and/or a computer program product.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 illustrates a diagram of an exemplary system in which one or more features of the disclosure subject matter can be implemented according to one or more embodiments;
FIG. 2 illustrates a block diagram of an example system for signal correction processing of velocity stream of a signal flow for coherent mapping of an organ according to one or more embodiments;
FIG. 3 illustrates an exemplary method according to one or more embodiments;
FIG. 4 illustrates a geometric diagram according to one or more embodiments;
FIG. 5 illustrates a geometric diagram according to one or more embodiments;
FIG. 6 illustrates geometric diagrams according to one or more embodiments;
FIG. 7 illustrates example interfaces according to one or more embodiments; and
FIG. 8 illustrates an exemplary method according to one or more embodiments.

DETAILED DESCRIPTION

Disclosed herein is a signal processing and method. More particularly, the present invention relates to signal correction processing of velocity streams of a signal flow for coherent mapping of an anatomical structure (or portion thereof), or any interpolation of vector fields on a surface (e.g., such as weather maps, wind velocity, air flow around a wing, earth's magnetic field at a surface for navigation, and like vortex flow descriptions in fluid mechanics). The signal correction processing is a processor executable code or software that is necessarily rooted in process operations by, and in processing hardware of, medical device equipment. For ease of explanation, the signal correction processing is described herein with respect to mapping at least a portion of anatomical structure, such as a human organ (e.g., a heart); however, any anatomical structure, body part, and/or organ or portion thereof can be a target for mapping by the signal and correction processing described herein. According to an exemplary embodiment, the signal correction processing is implemented by a mapping engine.

For instance, the mapping engine provides enhanced visualization that shows a flow or a progression of a complete velocity stream around an entire heart, such as by providing times, differences, and velocities and by verifying start and end points. In relation to the enhanced visualization of the flow (of a signal in a heart), especially to understand time and velocity for travel of the signal around the heart, particularly for cyclic arrhythmias, the mapping engine analyzes the signal along time intervals (e.g., 5 milliseconds or other intervals), as a measure of the velocity of the signal in time. Such an approach may be referred to as signal streams or velocity streams.

The advantages, technical effects, and benefits of the mapping engine may include at least providing cardiac physicians and medical personnel with a visualization of a flow of signal to verify a global correlation between the velocity vector solution and the LAT value solution. Thus, the mapping engine particularly utilizes and transforms medical device equipment to enable/implement enhanced visualizations that are otherwise not currently available or currently performed by cardiac physicians and medical personnel (e.g., as present cardiac physicians and medical personnel have a need to understand a flow of arrhythmias given that mesh forms of current medical mapping systems, when presenting any map, use an approximation to an anatomy based on a mesh).

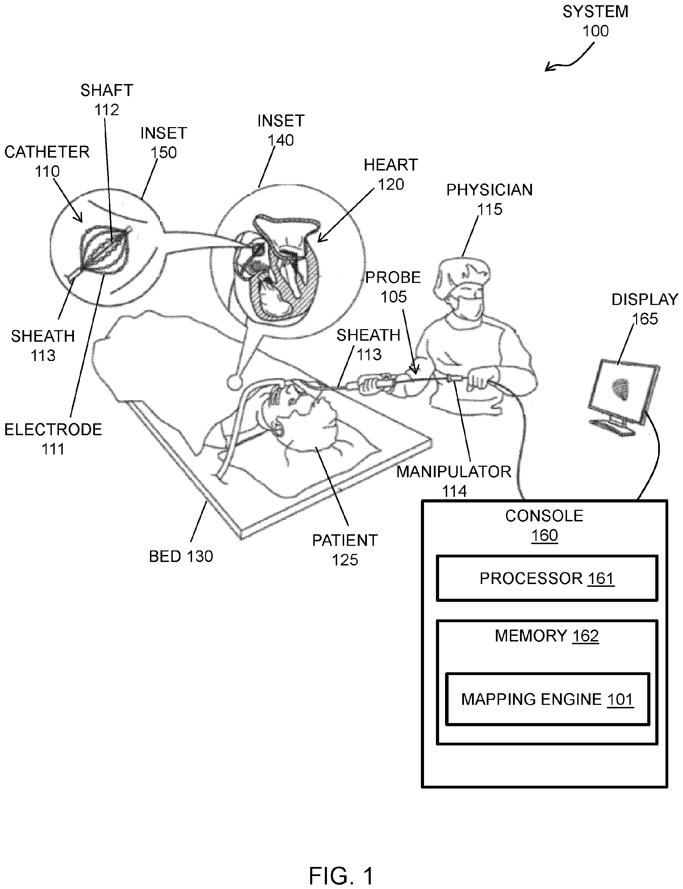

FIG. 1 is a diagram of a system 100 (e.g., medical device equipment) in which one or more features of the subject matter herein can be implemented according to one or more embodiments. All or part of the system 100 can be used to collect information (e.g., biometric data and/or a training dataset) and/or used to implement a machine learning and/or an artificial intelligence algorithm (e.g., a mapping engine 101) as described herein. The system 100, as illustrated, includes a probe 105 with a catheter 110 (including at least one electrode 111), a shaft 112, a sheath 113, and a manipulator 114. The system 100, as illustrated, also includes a physician 115 (or a medical professional or clinician), a heart 120, a patient 125, and a bed 130 (or a table). Note that insets 140 and 150 show the heart 120 and the catheter 110 in greater detail. The system 100 also, as illustrated, includes a console 160 (including one or more processors 161 and memories 162) and a display 165. Note further each element and/or item of the system 100 is representative of one or more of that element and/or that item. The example of the system 100 shown in FIG. 1 can be modified to implement the embodiments disclosed herein. The disclosed embodiments can similarly be applied using other system components and settings. Additionally, the system 100 can include additional components, such as elements for sensing electrical activity, wired or wireless connectors, processing and display devices, or the like.

The system 100 can be utilized to detect, diagnose, and/or treat cardiac conditions (e.g., using the mapping engine 101). Cardiac conditions, such as cardiac arrhythmias, persist as common and dangerous medical ailments, especially in the aging population. For instance, the system 100 can be part of a surgical system (e.g., CARTO® system sold by Biosense Webster) that is configured to obtain biometric data (e.g., anatomical and electrical measurements of a patient's organ, such as the heart 120) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 120. Such locating may be done via an electrophysiological investigation during which electrical potentials are detected spatially resolved with a mapping catheter (e.g., the catheter 110) introduced into the chamber of the heart 120. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. In this case, the mapping engine 101 can be directly stored and executed by the catheter 110.

In patients (e.g., the patient 125) with normal sinus rhythm (NSR), the heart (e.g., the heart 120), which includes atrial, ventricular, and excitatory conduction tissue, is electrically excited to beat in a synchronous, patterned fashion. Note that this electrical excitement can be detected as intracardiac electrocardiogram (IC ECG) data or the like.

In patients (e.g., the patient 125) with a cardiac arrhythmia (e.g., atrial fibrillation or aFib), abnormal regions of cardiac tissue do not follow a synchronous beating cycle associated with normally conductive tissue, which is in contrast to patients with NSR. Instead, the abnormal regions of cardiac tissue aberrantly conduct to adjacent tissue, thereby disrupting the cardiac cycle into an asynchronous cardiac rhythm. Note that this asynchronous cardiac rhythm can also be detected as the IC ECG data. Such abnormal conduction has been previously known to occur at various regions of the heart 120, for example, in the region of the sino-atrial (SA) node, along the conduction pathways of the atrioventricular (AV) node, or in the cardiac muscle tissue forming the walls of the ventricular and atrial cardiac chambers. There are other conditions, such as flutter, where the pattern of abnormally conducting tissues lead to reentry paths such that the chamber beats in a regular pattern that can be multiple times the sinus rhythm.

In support of the system 100 detecting, diagnosing, and/or treating cardiac conditions, the probe 105 can be navigated by the physician 115 into the heart 120 of the patient 125 lying on the bed 130. For instance, the physician 115 can insert the shaft 112 through the sheath 113, while manipulating a distal end of the shaft 112 using the manipulator 114 near the proximal end of the catheter 110 and/or deflection from the sheath 113. As shown in an inset 140, the catheter 110 can be fitted at the distal end of the shaft 112. The catheter 110 can be inserted through the sheath 113 in a collapsed state and can be then expanded within the heart 120.

Generally, electrical activity at a point in the heart 120 may be typically measured by advancing the catheter 110 containing an electrical sensor at or near its distal tip (e.g., the at least one electrode 111) to that point in the heart 120, contacting the tissue with the sensor and acquiring data at that point. One drawback with mapping a cardiac chamber using a catheter type containing only a single, distal tip electrode is the long period of time required to accumulate data on a point-by-point basis over the requisite number of points required for a detailed map of the chamber as a whole. Accordingly, multiple-electrode catheters (e.g., the catheter 110) have been developed to simultaneously measure electrical activity at multiple points in the heart chamber.

The catheter 110, which can include the at least one electrode 111 and a catheter needle coupled onto a body thereof, can be configured to obtain biometric data, such as electrical signals of an intra-body organ (e.g., the heart 120), and/or to ablate tissue areas of thereof (e.g., a cardiac chamber of the heart 120). Note that the electrodes 111 are representative of any like elements, such as tracking coils, piezoelectric transducer, electrodes, or combination of elements configured to ablate the tissue areas or to obtain the biometric data. According to one or more embodiments, the catheter 110 can include one or more position sensors that used are to determine trajectory information. The trajectory information can be used to infer motion characteristics, such as the contractility of the tissue.

Biometric data (e.g., patient biometrics, patient data, or patient biometric data) can include one or more of local time activations (LATs), electrical activity, topology, bipolar mapping, reference activity, ventricle activity, dominant frequency, impedance, or the like. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part.

Examples of biometric data include, but are not limited to, patient identification data, IC ECG data, bipolar intracardiac reference signals, anatomical and electrical measurements, trajectory information, body surface (BS) ECG data, historical data, brain biometrics, blood pressure data, ultrasound signals, radio signals, audio signals, a two- or three-dimensional image data, blood glucose data, and temperature data. The biometrics data can be used, generally, to monitor, diagnosis, and treatment any number of various diseases, such as cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

For example, the catheter 110 can use the electrodes 111 to implement intravascular ultrasound and/or MRI catheterization to image the heart 120 (e.g., obtain and process the biometric data). Inset 150 shows the catheter 110 in an enlarged view, inside a cardiac chamber of the heart 120. Although the catheter 110 is shown to be a point catheter, it will be understood that any shape that includes one or more electrodes 111 can be used to implement the embodiments disclosed herein.

Examples of the catheter 106 include, but are not limited to, a linear catheter with multiple electrodes, a balloon catheter including electrodes dispersed on multiple spines that shape the balloon, a lasso or loop catheter with multiple electrodes, or any other applicable shape. Linear catheters can be fully or partially elastic such that it can twist, bend, and or otherwise change its shape based on received signal and/or based on application of an external force (e.g., cardiac tissue) on the linear catheter. The balloon catheter can be designed such that when deployed into a patient's body, its electrodes can be held in intimate contact against an endocardial surface. As an example, a balloon catheter can be inserted into a lumen, such as a pulmonary vein (PV). The balloon catheter can be inserted into the PV in a deflated state, such that the balloon catheter does not occupy its maximum volume while being inserted into the PV. The balloon catheter can expand while inside the PV, such that those electrodes on the balloon catheter are in contact with an entire circular section of the PV. Such contact with an entire circular section of the PV, or any other lumen, can enable efficient imaging and/or ablation.

According to other examples, body patches and/or body surface electrodes may also be positioned on or proximate to a body of the patient 125. The catheter 110 with the one or more electrodes 111 can be positioned within the body (e.g., within the heart 120) and a position of the catheter 110 can be determined by the 100 system based on signals transmitted and received between the one or more electrodes 111 of the catheter 110 and the body patches and/or body surface electrodes. Additionally, the electrodes 111 can sense the biometric data from within the body of the patient 125, such as within the heart 120 (e.g., the electrodes 111 sense the electrical potential of the tissue in real time). The biometric data can be associated with the determined position of the catheter 110 such that a rendering of the patient's body part (e.g., the heart 120) can be displayed and show the biometric data overlaid on a shape of the body part.

The probe 105 and other items of the system 100 can be connected to the console 160. The console 160 can include any computing device, which employs the machine learning and/or an artificial intelligence algorithm (represented as the mapping engine 101). According to an embodiment, the console 160 includes the one or more processors 161 (any computing hardware) and the memory 162 (any non-transitory tangible media), where the one or more processors 161 execute computer instructions with respect the mapping engine 101 (e.g., a coherent mapping algorithm therein) and the memory 162 stores these instructions for execution by the one or more processors 161. For instance, the console 160 can be configured to receive and process the biometric data and determine if a given tissue area conducts electricity. In some embodiments, the console 160 can be further programmed by the mapping engine 101 (in software) to carry out the functions of subdividing an anatomical mesh of a part of an anatomical structure to one or more other meshes of the anatomical structure, interpolating local activation time values and velocity values for the one or more other meshes, tracing a path of velocity vectors on the one or more other meshes in accordance with the interpolation of the local activation time values and velocity values, and projecting the path on the anatomical mesh to provide an enhanced visualization of the anatomical structure. Note that the enhanced visualizations and the meshes, with respect to the heart, are usually of individual chambers, while the system 100 can display more than one chamber at a time. According to one or more embodiments, the mapping engine 101 can be external to the console 160 and can be located, for example, in the catheter 110, in an external device, in a mobile device, in a cloud-based device, or can be a standalone processor. In this regard, the mapping engine 101 can be transferable/downloaded in electronic form, over a network.

In an example, the console 160 can be any computing device, as noted herein, including software (e.g., the mapping engine 101) and/or hardware (e.g., the processor 161 and the memory 162), such as a general-purpose computer, with suitable front end and interface circuits for transmitting and receiving signals to and from the probe 105, as well as for controlling the other components of the system 100. For example, the front end and interface circuits include input/output (I/O) communication interfaces that enables the console 160 to receive signals from and/or transfer signals to the at least one electrode 111. The console 160 can include real-time noise reduction circuitry typically configured as a field programmable gate array (FPGA), followed by an analog-to-digital (A/D) ECG or electrocardiograph/electromyogram (EMG) signal conversion integrated circuit. The console 160 can pass the signal from an A/D ECG or EMG circuit to another processor and/or can be programmed to perform one or more functions disclosed herein.

The display 165, which can be any electronic device for the visual presentation of the biometric data, is connected to the console 160. According to an embodiment, during a procedure, the console 160 can facilitate on the display 165 a presentation of a body part rendering to the physician 115 and store data representing the body part rendering in the memory 162. For instance, maps depicting motion characteristics can be rendered/constructed based on the trajectory information sampled at a sufficient number of points in the heart 120. As an example, the display 165 can include a touchscreen that can be configured to accept inputs from the medical professional 115, in addition to presenting the body part rendering.

In some embodiments, the physician 115 can manipulate the elements of the system 100 and/or the body part rendering using one or more input devices, such as a touch pad, a mouse, a keyboard, a gesture recognition apparatus, or the like. For example, an input device can be used to change a position of the catheter 110, such that rendering is updated. Note that the display 165 can be located at a same location or a remote location, such as a separate hospital or in separate healthcare provider networks.

According to one or more embodiments, the system 100 can also obtain the biometric data using ultrasound, computed tomography (CT), MRI, or other medical imaging techniques utilizing the catheter 110 or other medical equipment. For instance, the system 100 can obtain ECG data and/or anatomical and electrical measurements of the heart 120 (e.g., the biometric data) using one or more catheters 110 or other sensors. More particularly, the console 160 can be connected, by a cable, to BS electrodes, which include adhesive skin patches affixed to the patient 125. The BS electrodes can procure/generate the biometric data in the form of the BS ECG data. For instance, the processor 161 can determine position coordinates of the catheter 110 inside the body part (e.g., the heart 120) of the patient 125. The position coordinates may be based on impedances or electromagnetic fields measured between the body surface electrodes and the electrode 111 of the catheter 110 or other electromagnetic components. Additionally, or alternatively, location pads, which generate magnetic fields used for navigation, may be located on a surface of the bed 130 and may be separate from the bed 130. The biometric data can be transmitted to the console 160 and stored in the memory 162. Alternatively, or in addition, the biometric data may be transmitted to a server, which may be local or remote, using a network as further described herein.

According to one or more embodiments, the catheter 110 may be configured to ablate tissue areas of a cardiac chamber of the heart 120. Inset 150 shows the catheter 110 in an enlarged view, inside a cardiac chamber of the heart 120. For instance, ablation electrodes, such as the at least one electrode 111, may be configured to provide energy to tissue areas of an intra-body organ (e.g., the heart 120). The energy may be thermal energy and may cause damage to the tissue area starting from the surface of the tissue area and extending into the thickness of the tissue area. The biometric data with respect to ablation procedures (e.g., ablation tissues, ablation locations, etc.) can be considered ablation data.

According to an example, with respect to obtaining the biometric data, a multi-electrode catheter (e.g., the catheter 110) can be advanced into a chamber of the heart 120. Anteroposterior (AP) and lateral fluorograms can be obtained to establish the position and orientation of each of the electrodes. ECGs can be recorded from each of the electrodes 111 in contact with a cardiac surface relative to a temporal reference, such as the onset of the P-wave in sinus rhythm from a BS ECG and/or the related to the signals from electrodes 111 of the catheter 110 placed in the coronary sinus. The system, as further disclosed herein, may differentiate between those electrodes that register electrical activity and those that do not due to absence of close proximity to the endocardial wall. After initial ECGs are recorded, the catheter may be repositioned, and fluorograms and ECGs may be recorded again. An electrical map (e.g., via cardiac mapping) can then be constructed from iterations of the process above.

Cardiac mapping can be implemented using one or more techniques. Generally, mapping of cardiac areas such as cardiac regions, tissue, veins, arteries and/or electrical pathways of the heart 120 may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping (which is an example of heart imaging) may include mapping based on one or more modalities such as, but not limited to local activation time (LAT), an electrical activity, a topology, a bipolar mapping, a dominant frequency, or an impedance. Data (e.g., biometric data) corresponding to multiple modalities may be captured using a catheter (e.g., the catheter 110) inserted into a patient's body and may be provided for rendering at the same time or at different times based on corresponding settings and/or preferences of the physician 115.

As an example of a first technique, cardiac mapping may be implemented by sensing an electrical property of heart tissue, for example, LAT, as a function of the precise location within the heart 120. The corresponding data (e.g., biometric data) may be acquired with one or more catheters (e.g., the catheter 110) that are advanced into the heart 1120 and that have electrical and location sensors (e.g., the electrodes 111) in their distal tips. As specific examples, location and electrical activity may be initially measured on about 10 to about 20 points on the interior surface of the heart 120. These data points may be generally sufficient to generate a preliminary reconstruction or map of the cardiac surface to a satisfactory quality. The preliminary map may be combined with data taken at additional points to generate a more comprehensive map of the heart's electrical activity. In clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation as described herein, to alter the propagation of the heart's electrical activity and to restore normal heart rhythm.

Further, cardiac mapping can be generated based on detection of intracardiac electrical potential fields (e.g., which is an example of IC ECG data and/or bipolar intracardiac reference signals). A non-contact technique to simultaneously acquire a large amount of cardiac electrical information may be implemented. For example, a catheter type having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes). As another more specific example, the catheter may include other multi-spline catheters, such as five soft flexible branches, eight radial splines, or a parallel splined pancake turner type (e.g., any of which may have a total of 42 electrodes).

As example of electrical or cardiac mapping, an electrophysiological cardiac mapping system and technique based on a non-contact and non-expanded multi-electrode catheter (e.g., the catheter 110) can be implemented. ECGs may be obtained with one or more catheters 110 having multiple electrodes (e.g., such as between 42 to 122 electrodes). According to this implementation, knowledge of the relative geometry of the probe and the endocardium can be obtained by an independent imaging modality, such as transesophageal echocardiography. After the independent imaging, non-contact electrodes may be used to measure cardiac surface potentials and construct maps therefrom (e.g., in some cases using bipolar intracardiac reference signals). This technique can include the following steps (after the independent imaging step): (a) measuring electrical potentials with a plurality of electrodes disposed on a probe positioned in the heart 120; (b) determining the geometric relationship of the probe surface and the endocardial surface and/or other reference; (c) generating a matrix of coefficients representing the geometric relationship of the probe surface and the endocardial surface; and (d) determining endocardial potentials based on the electrode potentials and the matrix of coefficients.

As another example of electrical or cardiac mapping, a technique and apparatus for mapping the electrical potential distribution of a heart chamber can be implemented. An intra-cardiac multi-electrode mapping catheter assembly can be inserted into the heart 120. The mapping catheter (e.g., the catheter 110) assembly can include a multi-electrode array with one or more integral reference electrodes (e.g., one or the electrodes 111) or a companion reference catheter.

According to one or more embodiments, the electrodes may be deployed in the form of a substantially spherical array, which may be spatially referenced to a point on the endocardial surface by the reference electrode or by the reference catheter this is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

In view of electrical or cardiac mapping and according to another example, the catheter 110 can be a heart mapping catheter assembly that may include an electrode array defining a number of electrode sites. The heart mapping catheter assembly can also include a lumen to accept a reference catheter having a distal tip electrode assembly that may be used to probe the heart wall. The map heart mapping catheter assembly can include a braid of insulated wires (e.g., having 24 to 64 wires in the braid), and each of the wires may be used to form electrode sites. The heart mapping catheter assembly may be readily positionable in the heart 120 to be used to acquire electrical activity information from a first set of non-contact electrode sites and/or a second set of in-contact electrode sites.

Further, according to another example, the catheter 110 that can implement mapping electrophysiological activity within the heart can include a distal tip that is adapted for delivery of a stimulating pulse for pacing the heart or an ablative electrode for ablating tissue in contact with the tip. This catheter 110 can further include at least one pair of orthogonal electrodes to generate a difference signal indicative of the local cardiac electrical activity adjacent the orthogonal electrodes.

As noted herein, the system 100 can be utilized to detect, diagnose, and/or treat cardiac conditions. In example operation, a process for measuring electrophysiologic data in a heart chamber may be implemented by the system 100. The process may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In preferred embodiments, the array is said to have from 60 to 64 electrodes.

As another example operation, cardiac mapping may be implemented by the system 100 using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of the probe 105 (e.g., a treatment catheter shown as catheter 110) at the later time may be displayed and the probe 105 may be overlaid onto the one or more ultrasound slices.

In view of the system 100, it is noted that cardiac arrhythmias, including atrial arrhythmias, may be of a multiwavelet reentrant type, characterized by multiple asynchronous loops of electrical impulses that are scattered about the atrial chamber and are often self-propagating (e.g., another example of the IC ECG data). Alternatively, or in addition to the multiwavelet reentrant type, cardiac arrhythmias may also have a focal origin, such as when an isolated region of tissue in an atrium fires autonomously in a rapid, repetitive fashion (e.g., another example of the IC ECG data). Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

For example, aFib occurs when the normal electrical impulses (e.g., another example of the IC ECG data) generated by the sinoatrial node are overwhelmed by disorganized electrical impulses (e.g., signal interference) that originate in the atria veins and PVs causing irregular impulses to be conducted to the ventricles. An irregular heartbeat results and may last from minutes to weeks, or even years. aFib is often a chronic condition that leads to a small increase in the risk of death often due to strokes. A line of treatment for aFib is medication that either slows the heart rate or revert the heart rhythm back to normal. Additionally, persons with aFib are often given anticoagulants to protect them from the risk of stroke. The use of such anticoagulants comes with its own risk of internal bleeding. In some patients, medication is not sufficient and their aFib is deemed to be drug-refractory, i.e., untreatable with standard pharmacological interventions. Synchronized electrical cardioversion may also be used to convert aFib to a normal heart rhythm. Alternatively, aFib patients are treated by catheter ablation.

A catheter ablation-based treatment may include mapping the electrical properties of heart tissue, especially the endocardium and the heart volume, and selectively ablating cardiac tissue by application of energy. Electrical or cardiac mapping (e.g., implemented by any electrophysiological cardiac mapping system and technique described herein) includes creating a map of electrical potentials (e.g., a voltage map) of the wave propagation along the heart tissue or a map of arrival times (e.g., a LAT map) to various tissue located points. Electrical or cardiac mapping (e.g., a cardiac map) may be used for detecting local heart tissue dysfunction. Ablations, such as those based on cardiac mapping, can cease or modify the propagation of unwanted electrical signals from one portion of the heart 120 to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. Another example of an energy delivery technique includes irreversible electroporation (IRE), which provides high electric fields that damage cell membranes. In a two-step procedure (e.g., mapping followed by ablation) electrical activity at points within the heart 120 is typically sensed and measured by advancing the catheter 110 containing one or more electrical sensors (e.g., electrodes 111) into the heart 120 and obtaining/acquiring data at a multiplicity of points (e.g., as biometric data generally, or as ECG data specifically). This ECG data is then utilized to select the endocardial target areas, at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems to reconstruct the anatomy of the heart chamber of interest. In this regard, the mapping engine 101 employed by the system 100 herein manipulates and evaluates the biometric data generally, or the ECG data specifically, to produce improved tissue data that enables more accurate diagnosis, images, scans, and/or maps for treating an abnormal heartbeat or arrhythmia. For example, cardiologists rely upon software, such as the Complex Fractionated Atrial Electrograms (CFAE) module of the CARTO® 3 3D mapping system, produced by Biosense Webster, Inc. (Diamond Bar, Calif.), to generate and analyze ECG data. The mapping engine 101 of the system 100 enhances this software to generate and analyze the improved biometric data, which further provide multiple pieces of information regarding electrophysiological properties of the heart 120 (including the scar tissue) that represent cardiac substrates (anatomical and functional) of aFib.

Accordingly, the system 100 can implement a 3D mapping system, such as CARTO® 3 3D mapping system, to localize the potential arrhythmogenic substrate of the cardiomyopathy in terms of abnormal ECG detection. The substrate linked to these cardiac conditions is related to the presence of fragmented and prolonged ECGs in the endocardial and/or epicardial layers of the ventricular chambers (right and left). In general, abnormal tissue is characterized by low-voltage ECGs. However, initial clinical experience in endo-epicardial mapping indicates that areas of low-voltage are not always present as the sole arrhythmogenic mechanism in such patients. In fact, areas of low or medium voltage may exhibit ECG fragmentation and prolonged activities during sinus rhythm, which corresponds to the critical isthmus identified during sustained and organized ventricular arrhythmias, e.g., applies only to non-tolerated ventricular tachycardias. Moreover, in many cases, ECG fragmentation and prolonged activities are observed in the regions showing a normal or near-normal voltage amplitude (>1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

As another example operation, cardiac mapping may be implemented by the system 100 using one or more multiple-electrode catheters (e.g., the catheter 110). Multiple-electrode catheters are used to stimulate and map electrical activity in the heart 120 and to ablate sites of aberrant electrical activity. In use, the multiple-electrode catheter is inserted into a major vein or artery, e.g., femoral vein, and then guided into the chamber of the heart 120 of concern. A typical ablation procedure involves the insertion of the catheter 110 having at least one electrode 111 at its distal end, into a heart chamber. A reference electrode is provided, taped to the skin of the patient or by means of a second catheter that is positioned in or near the heart or selected from one or the other electrodes 111 of the catheter 110. Radio frequency (RF) current is applied to a tip electrode 111 of the ablating catheter 110, and current flows through the media that surrounds it (e.g., blood and tissue) toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the tip electrode 111 also occurs as a result of conduction from the heated tissue to the electrode itself. If the electrode temperature becomes sufficiently high, possibly above 60 degrees Celsius, a thin transparent coating of dehydrated blood protein can form on the surface of the electrode 111. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter 110 must be removed from the body and the tip electrode 111 cleaned.

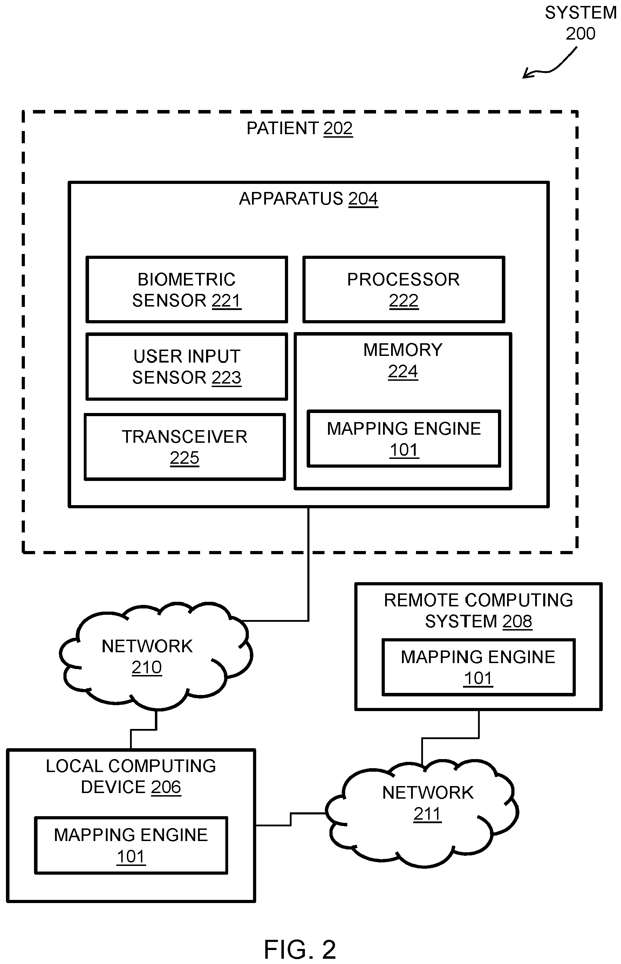

Turning now to FIG. 2, a diagram of a system 200 in which one or more features of the disclosure subject matter can be implemented is illustrated according to one or more embodiments. The system 200 includes, in relation to a patient 202 (e.g., an example of the patient 125 of FIG. 1), an apparatus 204, a local computing device 206, a remote computing system 208, a first network 210, and a second network 211. Further, the apparatus 204 can include a biometric sensor 221 (e.g., an example of the catheter 110 of FIG. 1), a processor 222, a user input (UI) sensor 223, a memory 224, and a transceiver 225. Note that the mapping engine 101 of FIG. 1 is reused in FIG. 2 for ease of explanation and brevity.

According to an embodiment, the apparatus 204 can be an example of the system 100 of FIG. 1, where the apparatus 204 can include both components that are internal to the patient and components that are external to the patient. According to an embodiment, the apparatus 204 can be an apparatus that is external to the patient 202 that includes an attachable patch (e.g., that attaches to a patient's skin). According to another embodiment, the apparatus 204 can be internal to a body of the patient 202 (e.g., subcutaneously implantable), where the apparatus 204 can be inserted into the patient 202 via any applicable manner including orally injecting, surgical insertion via a vein or artery, an endoscopic procedure, or a laparoscopic procedure. According to an embodiment, while a single apparatus 204 is shown in FIG. 2, example systems may include a plurality of apparatuses.

Accordingly, the apparatus 204, the local computing device 206, and/or the remote computing system 208 can be programed to execute computer instructions with respect the mapping engine 101. As an example, the memory 223 stores these instructions for execution by the processor 222 so that the apparatus 204 can receive and process the biometric data via the biometric sensor 201. IN this way, the processor 22 and the memory 223 are representative of processors and memories of the local computing device 206 and/or the remote computing system 208.

The apparatus 204, local computing device 206, and/or the remote computing system 208 can be any combination of software and/or hardware that individually or collectively store, execute, and implement the mapping engine 101 and functions thereof. Further, the apparatus 204, local computing device 206, and/or the remote computing system 208 can be an electronic, computer framework comprising and/or employing any number and combination of computing device and networks utilizing various communication technologies, as described herein. The apparatus 204, local computing device 206, and/or the remote computing system 208 can be easily scalable, extensible, and modular, with the ability to change to different services or reconfigure some features independently of others.

The networks 210 and 211 can be a wired network, a wireless network, or include one or more wired and wireless networks. According to an embodiment, the network 210 is an example of a short-range network (e.g., local area network (LAN), or personal area network (PAN)). Information can be sent, via the network 210, between the apparatus 204 and the local computing device 206 using any one of various short-range wireless communication protocols, such as Bluetooth, Wi-Fi, Zigbee, Z-Wave, near field communications (NFC), ultra-band, Zigbee, or infrared (IR). Further, the network 211 is an example of one or more of an Intranet, a local area network (LAN), a wide area network (WAN), a metropolitan area network (MAN), a direct connection or series of connections, a cellular telephone network, or any other network or medium capable of facilitating communication between the local computing device 206 and the remote computing system 208. Information can be sent, via the network 211, using any one of various long-range wireless communication protocols (e.g., TCP/IP, HTTP, 3G, 4G/LTE, or 5G/New Radio). Note that for either network 210 and 211 wired connections can be implemented using Ethernet, Universal Serial Bus (USB), RJ-11 or any other wired connection and wireless connections can be implemented using Wi-Fi, WiMAX, and Bluetooth, infrared, cellular networks, satellite or any other wireless connection methodology.

In operation, the apparatus 204 can continually or periodically obtain, monitor, store, process, and communicate via network 210 the biometric data associated with the patient 202. Further, the apparatus 204, local computing device 206, and/ the remote computing system 208 are in communication through the networks 210 and 211 (e.g., the local computing device 206 can be configured as a gateway between the apparatus 204 and the remote computing system 208). For instance, the apparatus 204 can be an example of the system 100 of FIG. 1 configured to communicate with the local computing device 206 via the network 210. The local computing device 206 can be, for example, a stationary/standalone device, a base station, a desktop/laptop computer, a smart phone, a smartwatch, a tablet, or other device configured to communicate with other devices via networks 211 and 210. The remote computing system 208, implemented as a physical server on or connected to the network 211 or as a virtual server in a public cloud computing provider (e.g., Amazon Web Services (AWS) ®) of the network 211, can be configured to communicate with the local computing device 206 via the network 211. Thus, the biometric data associated with the patient 202 can be communicated throughout the system 200.

Elements of the apparatus 224 are now described. The biometric sensor 221 may include, for example, one or more transducers configured to convert one or more environmental conditions into an electrical signal, such that different types of biometric data are observed/obtained/acquired. For example, the biometric sensor 221 can include one or more of an electrode (e.g., the electrode 111 of FIG. 1), a temperature sensor (e.g., thermocouple), a blood pressure sensor, a blood glucose sensor, a blood oxygen sensor, a pH sensor, an accelerometer, and a microphone.

The processor 222, in executing the mapping engine 101, can be configured to receive, process, and manage the biometric data acquired by the biometric sensor 221, and communicate the biometric data to the memory 224 for storage and/or across the network 210 via the transceiver 225. Biometric data from one or more other apparatuses 204 can also be received by the processor 222 through the transceiver 225. Also, as described in more detail herein, the processor 222 may be configured to respond selectively to different tapping patterns (e.g., a single tap or a double tap) received from the UI sensor 223, such that different tasks of a patch (e.g., acquisition, storing, or transmission of data) can be activated based on the detected pattern. In some embodiments, the processor 222 can generate audible feedback with respect to detecting a gesture.

The UI sensor 223 includes, for example, a piezoelectric sensor or a capacitive sensor configured to receive a user input, such as a tapping or touching. For example, the UI sensor 223 can be controlled to implement a capacitive coupling, in response to tapping or touching a surface of the apparatus 204 by the patient 202. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface, such that the tapping or touching of the surface activates the monitoring device.

The memory 224 is any non-transitory tangible media, such as magnetic, optical, or electronic memory (e.g., any suitable volatile and/or non-volatile memory, such as random-access memory or a hard disk drive). The memory 224 stores the computer instructions for execution by the processor 222.

The transceiver 225 may include a separate transmitter and a separate receiver. Alternatively, the transceiver 225 may include a transmitter and receiver integrated into a single device.

In operation, the apparatus 204, utilizing the mapping engine 101, observes/obtains the biometric data of the patient 202 via the biometric sensor 221, stores the biometric data in the memory, and shares this biometric data across the system 200 via the transceiver 225. The mapping engine 101 can then utilize any coherent mapping algorithm or combination thereof, such as fuzzy logic, models, neural networks, machine learning, and/or artificial intelligence to measure and track a flow and progression of signals around the heart and provide an enhanced visualization. For instance, the mapping engine 101 simplifies a chamber of the heart into a mesh of triangles, performs interpolation by calculation of individual velocity vectors for an electrical wave on the triangular mesh, and utilizes a whole map to visualize (e.g., provide an enhanced visualization of) the flow of the electrical signal over the entire heart chamber.

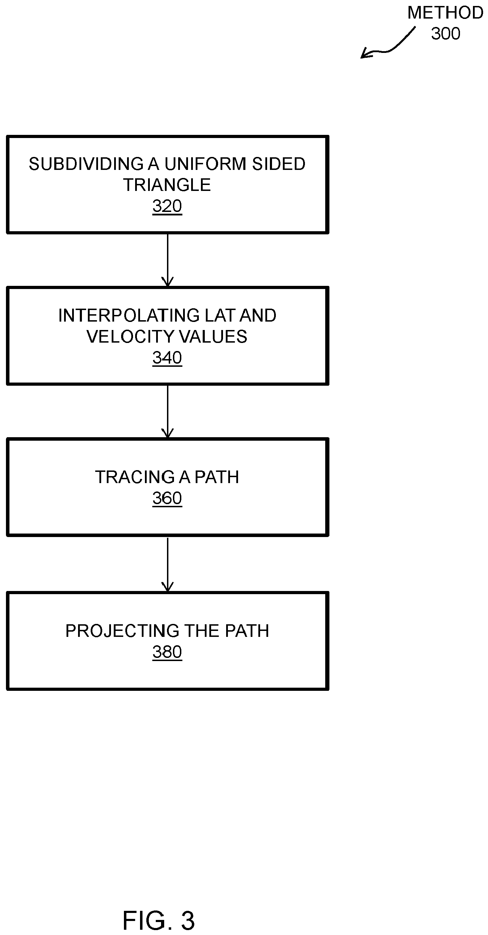

Turning now to FIGS. 3-6, mapping operations are described for the mapping engine 101.

With respect to FIG. 3, an exemplary method 300 (e.g., performed by the mapping engine 101 of FIG. 1 and/or of FIG. 2) is illustrated according to one or more exemplary embodiments. The exemplary method 300 addresses a need to understand a flow of arrhythmias given that mesh forms of current medical mapping systems, when presenting any map, for example LAT or voltage, use an approximation to the anatomy based on a mesh. The mesh may be detailed or coarse or have any combination of resolutions.

According to one or more embodiments, in addition to an original anatomical mesh, a secondary mesh can be used by the mapping engine 101. The mapping engine 101 uses the secondary mesh to approximate the original anatomical mesh. For instance, the secondary mesh is designed to have uniform sized triangles (e.g., of length 3.mm) and can be used as a basis of a coherent mapping algorithm to calculate the LAT and conduction velocity vectors, which are assigned to each triangle center (of the secondary mesh). Conventionally, when making a simple interpolation of these vectors and project them to the original anatomical mesh, a resulting image often looks discontinuous and correspondences between integrals of the time along these vectors does not correspond to the LAT difference along a trace. The mapping engine 101 overcomes this problem, as shown at least by the exemplary method 300.

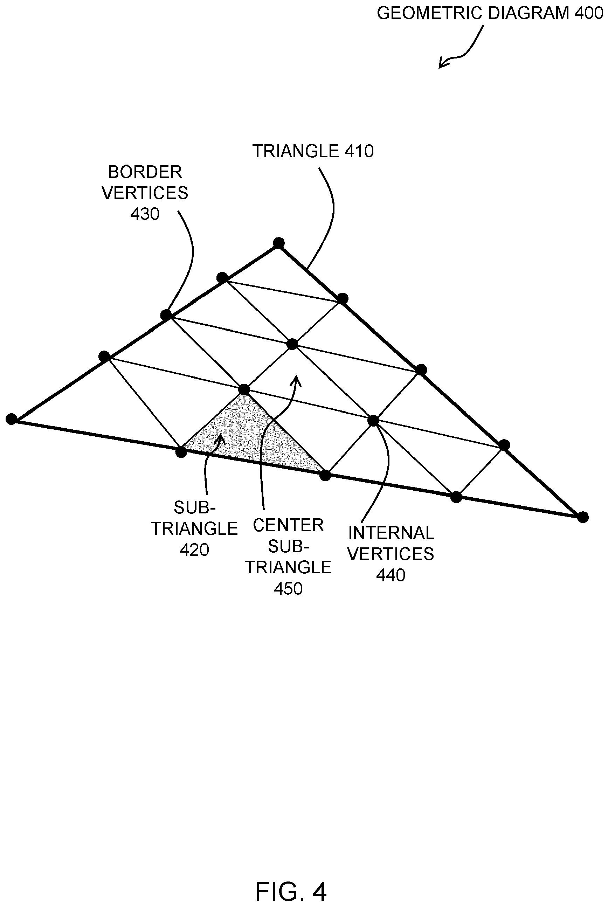

The exemplary method 300 begins at block 320, where the mapping engine 101 subdivides a uniform sided triangle mesh (e.g., an anatomical mesh or first mesh) to one or more finer meshes (e.g., one or more other or second meshes) of a heart chamber (e.g., at least a portion of an anatomical structure). For instance, FIG. 4 illustrates a geometric diagram 400 according to one or more embodiments, where the mapping engine 101 subdivides each existing/original triangle 410 into sixteen sub-triangles 420. Each triangle 410 (e.g., which contains single sub-triangles 420) constitutes a single plane. By sub-dividing the triangles 410, the mapping engine 101 has subdivided the anatomical mesh (e.g., a current or a simplified mesh) into the one or more other meshes (e.g., a more granular or a very fine mesh).

Further, the mapping engine 101 computes LAT values at vertices (e.g., border vertices 430 and internal vertices 440) using a center value and computes a velocity (e.g., from a coherent solution) to project to the vertices, with respect to Equation 1.

$$\mathrm{LAT}_{\mathrm{vertex}} = \mathrm{LAT}_{\mathrm{center}} + \vec{s}.\left(\vec{r}_{\mathrm{vertex}} - \vec{r}_{\mathrm{center}}\right) \qquad \text{Equation 1}$$

Note that, in some mathematical cases, the mapping engine 101 can have easier calculations working with slowness instead of velocity. Slowness is defined as a vector whose direction is identical to a velocity vector and whose magnitude is an inverse of the velocity vector magnitude, such that if v is velocity, define s for slowness as shown in Equation 2.

$$\vec{s} = \frac{\vec{v}}{\vec{v}\cdot\vec{v}} \qquad \text{Equation 2}$$

In one or more embodiments, if slowness is greater than 10, which indicates a very low conduction velocity, then the mapping engine 101 uses a vector with magnitude 10. Note that where the vertices belong to more than one existing/original triangle 410 (i.e., the border vertices 430), a mean value is taken. Also, LAT values at the vertices are used to compute velocity for each sub-triangle 420, and a mean of the LAT values at the internal vertices 440 is the LAT value at the center of each sub-triangle 420. A center sub-triangle 450 of the sixteen sub-triangles 420 preserves an original LAT and velocity vector.

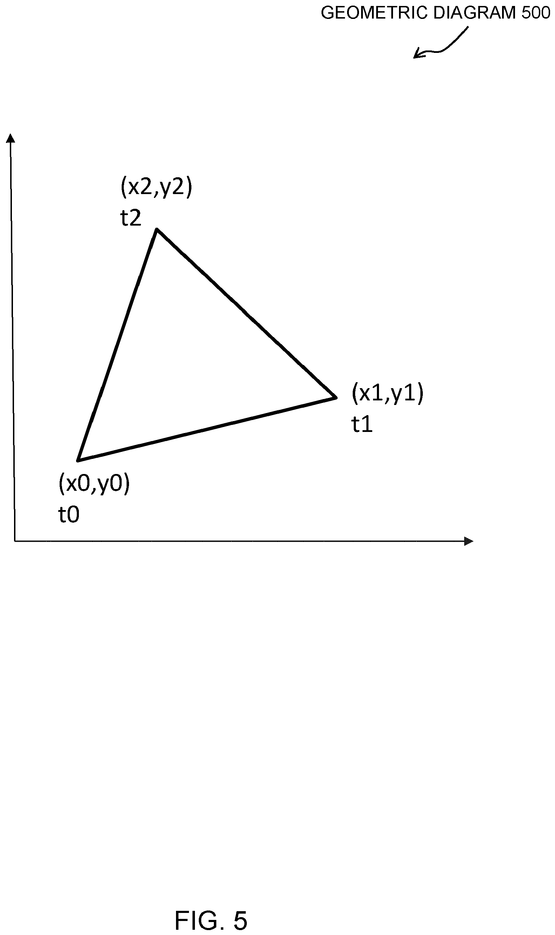

For instance, FIG. 5 illustrates a geometric diagram 500 according to one or more embodiments. The geometric diagram 500 illustrates a triangle for a slowness vector can be calculated. For instance, a coordinate transform is performed so that three vertices of the triangle are in an x-y plane of a new system. The vertices and slowness vector can now be represented as two-component vectors. As shown in FIG. 5 and by Equations 3 and 4.

$$(x1 - x0).(sx, sy) = (t1 - t0) \qquad \text{Equation 3}$$

$$(x2 - x0).(sx, sy) = (t2 - t0) \qquad \text{Equation 4}$$

Further, in terms of velocity, a solution of the Equations 4 and 5 yields the explicit Equation 5, which can be transformed back into the original three dimensional coordinate system.

$$\begin{pmatrix} \mathrm{velplane}(1) \\ \mathrm{velplane}(2) \end{pmatrix} = \begin{pmatrix} \dfrac{(t2\,y1 - t1\,y2)\,(x2\,y1 - x1\,y2)}{t2^2 x1^2 - 2t1\,t2\,x1\,x2 + t1^2\,x2^2 + t2^2 y1^2 - 2t1\,t2\,y1\,y2 + t1^2\,y2^2} \\[2ex] \dfrac{-(t2\,x1 - t1\,x2)(x2\,y1 - x1\,y2)}{t2^2 x1^2 - 2t1\,t2\,x1\,x2 + t1^2\,x2^2 + t2^2 y1^2 - 2t1\,t2\,y1\,y2 + t1^2\,y2^2} \end{pmatrix}$$

$$\text{Equation 5}$$

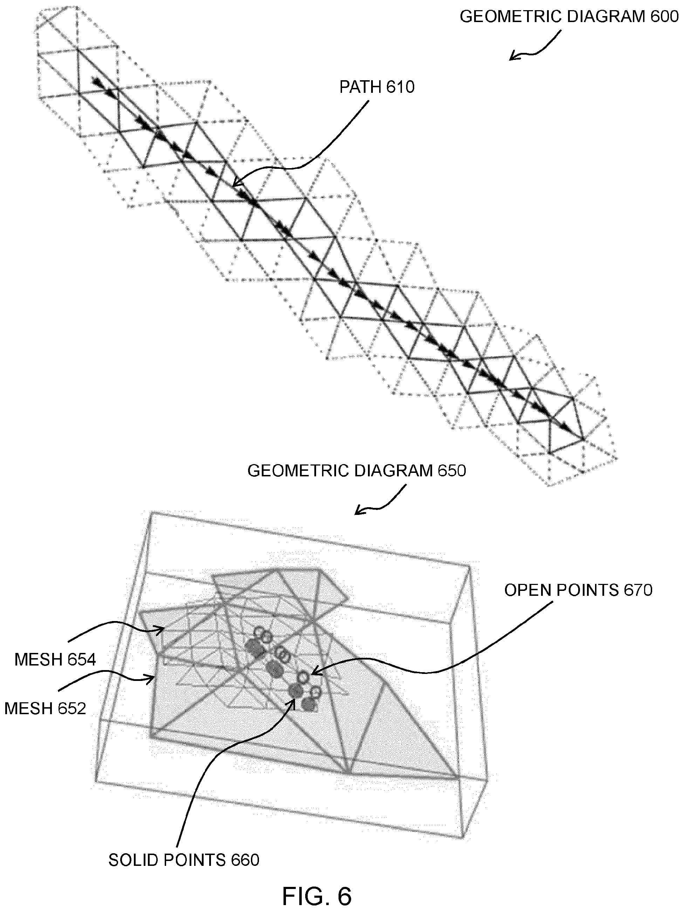

At block 340, the mapping engine 101 interpolates LAT and velocity values for the very fine mesh. At block 360, the mapping engine 101 traces a path of velocity vectors in accordance with the interpolations. For instance, FIG. 6 illustrates geometric diagrams 600 and 650 according to one or more embodiments, where the mapping algorithm (e.g., a coherent mapping algorithm of the mapping engine 101) performs interpolation of LAT and velocity values to trace a path 610 of the velocity vectors (e.g., an internal path). Further, the mapping engine 101 receives an anatomical mesh (e.g., the geometric diagram 650, which is drawn by physician 115 or the like) and traces the catheter 110 along an inner surface to create a triangular mesh that approximates the anatomy. Note that, in the geometric diagram 650, a mesh 652 is an example of a CARTO anatomic mesh, where triangles therein are less equilateral than in a mesh 654. The mesh 654 is a "coarse" mesh used in the coherent mapping algorithm, with each triangle divided by 16. Note that the mesh 654 is a simplification, sort of a thinning out of an original CARO anatomical mesh, so that the projections are very well behaved. The solid points 660 are uniform steps in a time tracing on the mesh 654 (e.g., .1 msec.). The open points 670 are projections to the CARTO mesh (e.g., a display is a connection of the green points).

Note that, according to one or more embodiments, a coarse mesh is a mesh on which the coherent mapping algorithm supplies the LAT values and velocity vectors and is not seen by a user (e.g., physician 115). Rather, the user sees LAT values and velocity vectors projected to the CARTO anatomical mesh that, depending on the case, may be finer or coarser. In an example, the internal "coarse" mesh of coherent mapping algorithm is designed to have an average face length of 3.5mm. In turn, a working mesh for the interpolation scheme can have an average face length of 3.5/4

(~.9) mm. If the original velocity vectors from the coherent mapping algorithm were only presented at the projection to the anatomical of the locations where they are calculated, the view would look a sparse or disjointed. Thus, conventionally, a simple interpolation of the vectors are displayed (e.g., in the field, resulting velocity vector streams looked wrong/funny, and the vectors did not look like what would be expected from the gradient of the times). The mapping engine 101 solves this concern by interpolating the velocity vectors inside and between the coherent triangles in a mathematically correct way so that the flow appears continuous and the integration over the velocity path match the time difference (e.g., LAT) from beginning to end of the path. When these points are projected to the anatomical mesh, the vectors look good and are mathematically correct.

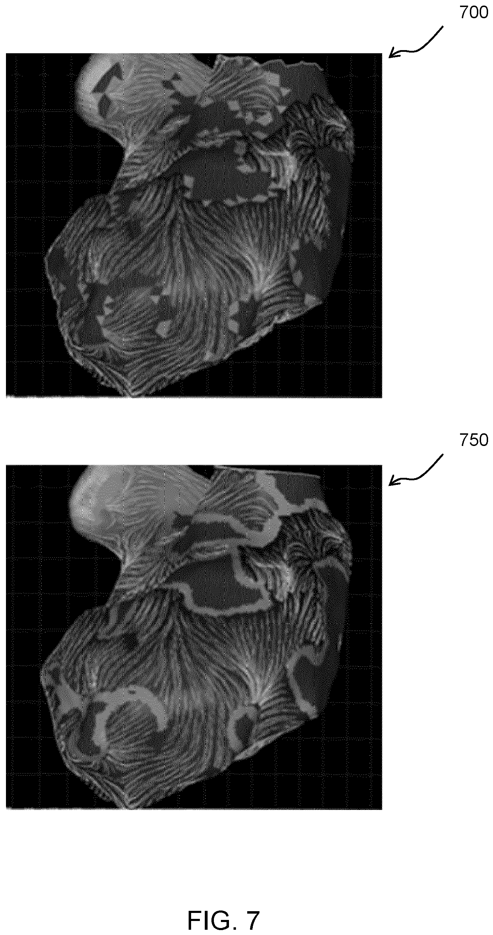

At block 380, the mapping engine 101 projects the path on the simplified mesh to provide an enhanced visualization of the heart. In one or more exemplary embodiments, the mapping engine 101 projects each point in a stream orthogonally to a mesh. Particularly, as shown in FIG. 7, example interfaces 700 and 750 show the projections of points in the stream orthogonally to the CARTO mesh, where the interface 700 is a simplified mesh and the interface 750 is a very fine mesh. Note that if a point is on an edge where two triangles have different normal vectors, the mapping system 101 uses an average direction of the two different normal vectors. The interface 700 and the interface 750 tend to overlap nicely, except near openings where the interface 750 has less coverage.

The advantages, technical effects, and benefits of the method 300 at least include providing cardiac physicians and medical personnel with a visualization of a flow of signal to verify a global correlation between the velocity vector solution and the LAT value solution. Thus, the mapping engine 101 particularly utilizes and transforms medical device equipment to enable/implement enhanced visualizations that are otherwise not currently available or currently performed by cardiac physicians and medical personnel.

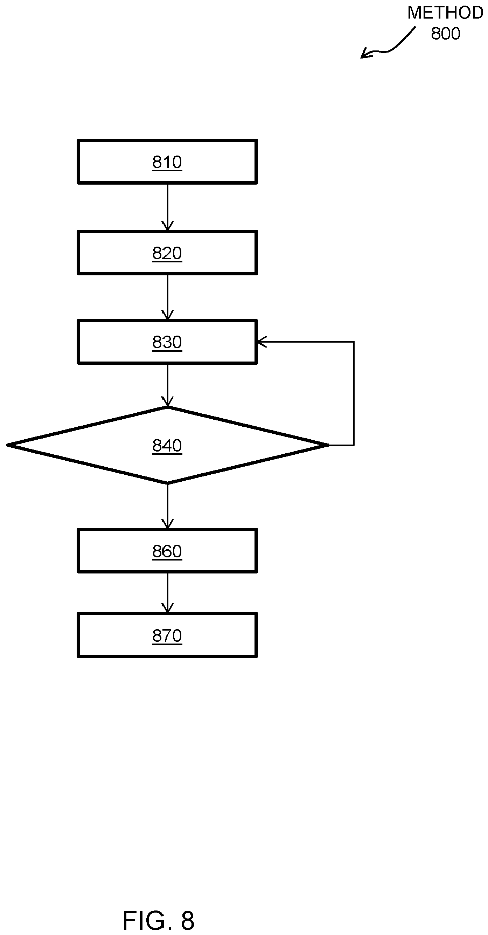

Turning to FIG. 8, an exemplary method 800 is shown according to one or more embodiments. The exemplary method 800 (e.g., performed by the mapping engine 101 of FIG. 1 and/or of FIG. 2) is illustrated according to one or more exemplary embodiments. The exemplary method 800 addresses a need to understand a flow of arrhythmias given that mesh forms of current medical mapping systems are coarse by providing a multi-step manipulation of electrical signals that enables an improved understanding an electrophysiology with more precision. That is, in general, the mapping engine 101 by implementing the exemplary method 800 takes a given signal origination and, then, calculates a next sub-triangle into which the signal moves. Because each sub-triangle of a mesh corresponds to a certain time and velocity, the mapping engine 101 interpolates the LATs and velocity values for each sub-triangle into which the signal moves. The mapping engine 101 projects from one sub-triangle to the next, over short units of time, to trace the complete progress and whole path of the signal.

More particularly, the exemplary method begins at block 810, where the mapping engine 101 locates an initial signal orientation within a middle portion of a sub-triangle. At block 820, a uniform time interval for steps is chosen dt (e.g., for example .1 msec.). For instance, it can be perceived that a particle is flowing along with starting point at a center of an initial triangle (e.g., the sub-triangle).

At block 830, a location of the particle is determined. For instance, the location of the particle after the first step determined according to Equation 6.

$$r_1 = r_0 + dt\vec{v} \qquad\qquad Equation\ 6$$

At determination block 840, the location is repeated checked as to whether a border (e.g., seam) of the initial triangle is crossed (e.g., using Plucker coordinates). Note that at a border the path is continued for the remainder of the time step using the velocity of the adjacent triangle.

In accordance with one or more embodiments, with respect to the edge of the sub-triangle, the mapping engine 101 addresses when an early seam meets a late seam. That is, when an early boundary meets late boundary, the change in LAT (e.g., ∆LAT) is very large and the velocity is unphysically low. In turn, the mapping engine calculate a cyclic shifted LAT by using shifted LAT values to perform subdivision and vertex LAT calculations and calculating the velocities for each small triangle. Note that the velocities may be the same except at the early seam/boundary meets late seam/boundary. Further, the mapping engine 101 repeats the subdivision of the faces and velocity calculations using the shifted LATs. For velocity vectors in the stream calculations, the mapping engine 101 chooses a larger of the two velocities. Near early meets late, small vertex LATs can be widely different. For small faces with vertices having larger LAT differences (e.g., LAT differences > 10), the mapping engine 101 replaces the LAT value with the cyclic shifted interpolated LAT cyclic shifted back.

The mapping engine 101 arranges the inter-connected sub-triangles in a three-dimensional space, so the signal path can change planes as the signal path progresses around the heart, from sub-triangle to sub-triangle. Note that the interpolation process keeps each velocity in its own plane.

The mapping engine 101 provides the velocity streams on the subdivided mesh. In this regard, the mapping engine 101 starts at center of a triangle and follows a velocity vector to the edge of the triangle. Note that within a small triangle, velocity can be constant. The mapping engine 101, then, provides a LAT at edge as an interpolated value of the two vertices. The mapping engine 101 can further loop through determining which triangle is next, following the velocity vector to the edge of the triangle, and tracking a total sum according to Equation 7.

$$\sum_i \overrightarrow{segment_i} \cdot \overrightarrow{slowness_i} \qquad \text{Equation 7}$$

The loop can stop when the mapping engine 101 hits a scar and/or when a desired elapsed time (e.g., interval of 5 milliseconds or other value) is reached.

The mapping engine 101 can testing for intersections, such as by using coordinates (e.g., Plucker coordinates can be used by the mapping engine 101 to assign six homogeneous coordinates to each line in projective space). For instance, Equation 8 can be applied for two points (x1,y1,z1) and (x2,y2,z2) on a line (e.g., L) and a side operator can be used for lines a and b according to Equation 9.

$$L = \{x1y2 - x2y1, x1z2 - x2z1, y1z2 - y2z1, z1 - z2, y2 - y1\}$$

$$\text{Equation 8}$$

$$S(a, b) = a1b5 + a2b6 + a3b4 + a4b3 + a5b1 + a6b2 \qquad \text{Equation 9}$$

Further, the mapping engine 101 considers any point P that is not in the plane formed by line segment S and the line L. Then, if S1 and S2 have different signs (e.g., one < 0 and other > 0), then the line L does not intersect the line segment S; if S1 and S2 have same sign ( > 0 or < 0), then the line L passes through the line segment S; and if S1 = S2 = 0, then the line L contains the line segment S. Note that if one out of s1, s2 is zero, then the line L passes through an end point of the line segment S.

At block 860, the method 800 is continued until the total time reaches a predetermined limit (e.g., such as 10 msec.), or the test particle reaches a mesh border triangle or a triangle which was determined to be non-conducting. The total time is calculated by summing over each segment, according to Equation 10.

$$T_{total} = \sum_i \vec{s}_i \cdot \left( \vec{r}_i - \vec{r}_{i-1} \right) \qquad \text{Equation 10}$$

In many cases, the LAT values can be cyclic. In these cases, the LAT values correspond to phases of a cardiac cycle so that an earliest and latest times are essentially the same and the signal flows smoothly across the early meets late border.

At block 870, the mapping engine 101 projects a path calculated on the fine mesh to the system anatomical mesh to provide an enhanced visualization of the heart. In one or more exemplary embodiments, the mapping engine 101 projects each point in a stream orthogonally to a mesh.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

**Claims**

1. A method comprising:

   subdividing, by a mapping engine executed by one or more processors, an anatomical mesh of at least a part of an anatomical structure to one or more other meshes of the anatomical structure, the one or more other meshes being more granular than the anatomical mesh;
   interpolating, by the mapping engine, local activation time values and velocity values for the one or more other meshes;
   tracing, by the mapping engine, a path of velocity vectors on the one or more other meshes in accordance with the interpolation of the local activation time values and velocity values; and
   projecting, by the mapping engine, the path on the anatomical mesh to provide an enhanced visualization of the anatomical structure.

2. A system comprising:

   a memory storing program code for a mapping engine;
   at least one processor executing the program code to cause the system to perform:

   subdividing, by the mapping engine, an anatomical mesh of at least a part of an anatomical structure to one or more other meshes of the anatomical structure, the one or more other meshes being more granular than the anatomical mesh;
   interpolating, by the mapping engine, local activation time values and velocity values for the one or more other meshes;
   tracing, by the mapping engine, a path of velocity vectors on the one or more other meshes in accordance with the interpolation of the local activation time values and velocity values; and
   projecting, by the mapping engine, the path on the anatomical mesh to provide an enhanced visualization of the anatomical structure.

3. The method of claim 1 or the system of claim 2, wherein the anatomical mesh comprises at least one triangle and the one or more other meshes comprises a plurality of sub-triangles in the at least one triangle.

4. The method or system of claim 3, wherein each of the at least one triangle constitutes a single plane for the anatomical mesh.

5. The method or system of claim 3, wherein the mapping engine determines the local activation time values at vertices of the plurality of sub-triangles using a center value.

6.  The method or system of claim 5, wherein the mapping engine determines a velocity to project to the vertices.

7.  The method or system of claim 5, wherein the vertices belong to more than one of the at least one triangle, a mean value is taken for the local activation time values.

8.  The method or system of claim 5, wherein the local activation time values at the vertices are used by the mapping engine to compute the velocity values for each of the plurality of sub-triangles.

9.  The method of claim 1 or the system of claim 2, wherein the mapping engine subdivides each of the at least one triangle into sixteen sub-triangles.

10. The method of claim 1 or the system of claim 2, wherein a center sub-triangle of the plurality of sub-triangles preserves an original local activation time and velocity vector.

11. The method of claim 1 or the system of claim 2, wherein the anatomical structure comprises a heart, and the at least part of the anatomical structure comprises a chamber.

FIG. 1

SYSTEM
200

PATIENT 202

APPARATUS 204

| BIOMETRIC SENSOR 221 | PROCESSOR 222 |
|---|---|
| USER INPUT SENSOR 223 | MEMORY 224 |
| TRANSCEIVER 225 | MAPPING ENGINE 101 |

NETWORK
210

REMOTE COMPUTING
SYSTEM 208

MAPPING ENGINE
101

LOCAL COMPUTING
DEVICE 206

MAPPING ENGINE
101

NETWORK
211

FIG. 2

METHOD
300

```
┌─────────────────────────────┐
│  SUBDIVIDING A UNIFORM SIDED │
│           TRIANGLE           │
│             320              │
└─────────────────────────────┘
              │
              ▼
┌─────────────────────────────┐
│    INTERPOLATING LAT AND     │
│       VELOCITY VALUES        │
│             340              │
└─────────────────────────────┘
              │
              ▼
┌─────────────────────────────┐
│        TRACING A PATH        │
│             360              │
└─────────────────────────────┘
              │
              ▼
┌─────────────────────────────┐
│      PROJECTING THE PATH     │
│             380              │
└─────────────────────────────┘
```

FIG. 3

GEOMETRIC DIAGRAM 400

TRIANGLE 410

BORDER
VERTICES
430

SUB-
TRIANGLE
420

CENTER
SUB-
TRIANGLE
450

INTERNAL
VERTICES
440

FIG. 4

GEOMETRIC DIAGRAM 500

FIG. 5

GEOMETRIC DIAGRAM 600

PATH 610

GEOMETRIC DIAGRAM 650

OPEN POINTS 670

MESH 654

MESH 652

SOLID POINTS 660

FIG. 6

700

750

FIG. 7

METHOD
800

```
        ┌──────────────────┐
        │        810        │
        └──────────────────┘
                 │
                 ▼
        ┌──────────────────┐
        │        820        │
        └──────────────────┘
                 │
                 ▼
        ┌──────────────────┐
        │        830        │◄────────┐
        └──────────────────┘         │
                 │                    │
                 ▼                    │
              ╱──────╲                │
            ╱    840    ╲─────────────┘
            ╲          ╱
              ╲──────╱
                 │
                 ▼
        ┌──────────────────┐
        │        860        │
        └──────────────────┘
                 │
                 ▼
        ┌──────────────────┐
        │        870        │
        └──────────────────┘
```

FIG. 8

Europäisches
Patentamt

European
Patent Office

Office européen
des brevets

# EUROPEAN SEARCH REPORT

Application Number

EP 21 21 2677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAM COVENEY ET AL: "Gaussian Process Manifold Interpolation for Probabilistic Atrial Activation Maps and Uncertain Conduction Velocity", ARXIV.ORG, CORNELL UNIVERSITY LIBRARY, 201 OLIN LIBRARY CORNELL UNIVERSITY ITHACA, NY 14853, 22 April 2020 (2020-04-22), XP081650441, * figures 1,2,3,4,7 * * section 1 "background", paragraph 4, last 5 lines, page 2 * * section 1 "background", last paragraph, lines 1 to 5, page 2 * * section 2.1 "data collection" * * section 2.5 "eigenfunction calculations", page 5 last paragraph to page 6, first paragraph, first line * * section 2.6 "conduction velocity distribution", first paragraph, lines 1 to 5, page 6 * * section 2.7 "simulations", paragraphs 2 and 3, page 7 * * section 3.1 "LAT interpolation", paragraph 1, page 8 * * section 3.2 "conduction velocity", first paragraph, bridging pages 8 and 9 * * section 3.2 "conduction velocity", page 11, second paragraph, lines 4 to 6 * ----- -/-- | 1-11 | INV. G06T17/20 G06T19/00  TECHNICAL FIELDS SEARCHED (IPC)  G06T |

The present search report has been drawn up for all claims

| Place of search | Date of completion of the search | Examiner |
|---|---|---|
| Berlin | 18 May 2022 | Gauthier, J |

EPO FORM 1503 03.82 (P04C01)

Europäisches
Patentamt

European
Patent Office

Office européen
des brevets

# EUROPEAN SEARCH REPORT

Application Number

EP 21 21 2677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JIANHUA LI ET AL: "An improved subdivision algorithm using for heart model", AUDIO, LANGUAGE AND IMAGE PROCESSING (ICALIP), 2012 INTERNATIONAL CONFERENCE ON, IEEE, 16 July 2012 (2012-07-16), pages 346-350, XP032277979, DOI: 10.1109/ICALIP.2012.6376639 ISBN: 978-1-4673-0173-2 * figure 2 * * section 2 "Loop algorithm and 1-16 grid subdivision", pages 346 to 347 * * section 3 "the results and analysis", subsection 31. "the 1-16 subdivision", page 348 * | 9 | |
| A | US 2019/259490 A1 (COHEN BENJAMIN [IL] ET AL) 22 August 2019 (2019-08-22) * paragraph [0004] – paragraph [0005] * * paragraph [0010] * * paragraph [0013] * * paragraph [0024] – paragraph [0026] * * paragraph [0031] – paragraph [0032] * * paragraph [0036] * * paragraph [0040] * | 1-11 | TECHNICAL FIELDS SEARCHED (IPC) |
| A | US 2019/059766 A1 (HOUBEN RICHARD P M [BE] ET AL) 28 February 2019 (2019-02-28) * paragraph [0017] – paragraph [0019] * * paragraph [0093] * * paragraph [0121] – paragraph [0135] * * paragraph [0153] – paragraph [0155] * | 1-11 | |

-/--

The present search report has been drawn up for all claims

| Place of search | Date of completion of the search | Examiner |
|---|---|---|
| Berlin | 18 May 2022 | Gauthier, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 21 21 2677**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/125383 A1 (HAN DONGFENG [US] ET AL) 10 May 2018 (2018-05-10)<br>* paragraph [0003] *<br>* paragraph [0006] *<br>* paragraph [0021] – paragraph [0022] *<br>* paragraph [0049] *<br>* paragraph [0070] *<br>* paragraph [0114] *<br>————— | 1–11 | |

| | | | |
|---|---|---|---|
| | | | TECHNICAL FIELDS SEARCHED (IPC) |

The present search report has been drawn up for all claims

| Place of search | Date of completion of the search | Examiner |
|---|---|---|
| Berlin | 18 May 2022 | Gauthier, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 2677

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2022

| Patent document cited in search report | | Publication date | Patent family member(s) | | Publication date |
|---|---|---|---|---|---|
| US 2019259490 | A1 | 22-08-2019 | AU | 2019201271 A1 | 05-09-2019 |
| | | | CA | 3034368 A1 | 22-08-2019 |
| | | | CN | 110179458 A | 30-08-2019 |
| | | | EP | 3530184 A1 | 28-08-2019 |
| | | | JP | 2019177135 A | 17-10-2019 |
| | | | US | 2019259490 A1 | 22-08-2019 |
| US 2019059766 | A1 | 28-02-2019 | AU | 2017201829 A1 | 19-10-2017 |
| | | | CA | 2961626 A1 | 30-09-2017 |
| | | | CN | 107260157 A | 20-10-2017 |
| | | | EP | 3225161 A1 | 04-10-2017 |
| | | | ES | 2748629 T3 | 17-03-2020 |
| | | | IL | 251312 A | 31-08-2020 |
| | | | IL | 275975 A | 30-11-2020 |
| | | | JP | 6866207 B2 | 28-04-2021 |
| | | | JP | 2017185223 A | 12-10-2017 |
| | | | US | 2017281031 A1 | 05-10-2017 |
| | | | US | 2019059766 A1 | 28-02-2019 |
| | | | US | 2020297234 A1 | 24-09-2020 |
| US 2018125383 | A1 | 10-05-2018 | CN | 108348155 A | 31-07-2018 |
| | | | EP | 3344124 A1 | 11-07-2018 |
| | | | JP | 6445739 B2 | 26-12-2018 |
| | | | JP | 2018526107 A | 13-09-2018 |
| | | | US | 2017055864 A1 | 02-03-2017 |
| | | | US | 2018125383 A1 | 10-05-2018 |
| | | | US | 2020281491 A1 | 10-09-2020 |
| | | | WO | 2017040581 A1 | 09-03-2017 |

EPO FORM P0459

For more details about this annex : see Official Journal of the European Patent Office, No. 12/82

**REFERENCES CITED IN THE DESCRIPTION**

**Patent documents cited in the description**

- US 20200146579 A